(19) Europäisches Patentamt
European Patent Office
Office européen des brevets

(11) Veröffentlichungsnummer: **0 176 773**
**B1**

(12) # EUROPÄISCHE PATENTSCHRIFT

(45) Veröffentlichungstag der Patentschrift: **27.12.89**

(51) Int. Cl.⁴: **A 61 K 31/44,** A 61 K 9/22

(21) Anmeldenummer: **85110921.5**

(22) Anmeldetag: **30.08.85**

(54) **Feste Arzneizubereitung enthaltend Nitrendipin und Verfahren zur ihrer Herstellung.**

(30) Priorität: **11.09.84 DE 3433239**

(43) Veröffentlichungstag der Anmeldung:
**09.04.86 Patentblatt 86/15**

(45) Bekanntmachung des Hinweises auf die Patenterteilung:
**27.12.89 Patentblatt 89/52**

(84) Benannte Vertragsstaaten:
**AT BE CH DE FR GB IT LI NL SE**

(56) Entgegenhaltungen:
**FR-A-2 524 314**

**CHEMICAL ABSTRACTS, Band 75, Nr. 20, 15. November 1971, Seite 215, Nr. 121361a, Columbus, Ohio, US; E.E. BORZUNOV: "Sodium lauryl sulfate, a hydrophilizing auxiliary substance in tablet production" & FARM. ZH. (KIEV) 1971, 26(4), 49-52**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Schmidt, Wolfgang, Bilharzstrasse 3, D-5000 Köln 80 (DE)**
Erfinder: **Streuff, Bernhard, Dr., Asterweg 23, D-5632 Wermelskirchen 2 (DE)**
Erfinder: **Winter, Manfred, Dr., Roggendorfstrasse 49, D-5000 Köln 80 (DE)**

**EP 0 176 773 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft eine spezielle, gut resorbierbare feste Arzneizubereitung, die kristallines Nitrendipin, Polyvinylpyrrolidon und Natriumlaurylsulfat enthält sowie Verfahren zu ihrer Herstellung.

Die Verbindung Nitrendipin (2,6-Dimethyl-4-(3'-nitro-phenyl)-1,4-dihydropyridin-3,5-di-carbonsäure-3-methyl-5-ethylester) der Formel

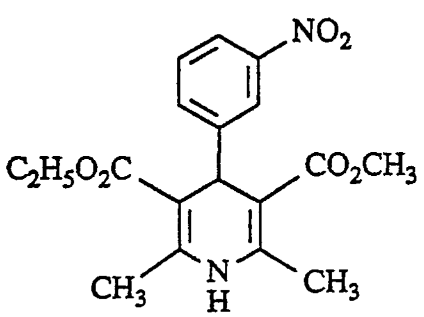

kann hergestellt werden durch Umsetzung des Yliden-β-Ketocarbonsäureesters der Formel

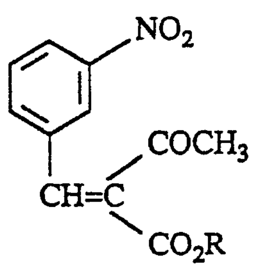

in der
R Methyl oder Ethyl darstellt mit einem Enaminocarbonsäureester der allgemeinen Formel

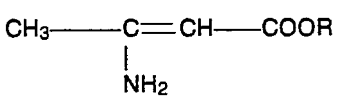

in der
R Methyl oder Ethyl bedeutet, oder durch Umsetzung des obigen Yliden-β-Ketocarbonsäureesters mit Ammoniak und einer Ketocarbonsäure der Formel

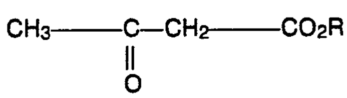

in der
R Methyl oder Ethyl darstellt.

Die Verbindung Nitrendipin und ihre blutdrucksenkende Wirkung ist bereits bekannt (Britisches Patent Nr. 1 358 951). Spezielle galenische Formulierungen, welche Nitrendipin in Kopräzipitatform (nicht kristallin) enthalten, sind ebenso bekannt (DE-OS-3 142 853).

In dem französischen Patent Nr. 8 305 518 (Publikationsnummer 2 524 314) sind bereits Arzneizubereitungen beschrieben, die neben anderen Hilfsstoffen das Detergenz Tween 80® enthalten. Vergleichende Untersuchungen mit den erfindungsgemäßen Zubereitungen zeigen jedoch, daß Nitrendipin-Tabletten mit Tween 80® bezüglich ihrer Freisetzung, ihrer mechanischen Stabilität und ihrer Größe den erfindungsgemäßen Nitrendipin-Zubereitungen, die das Detergenz Natriumlaurylsulfat enthalten, unterlegen sind.

Aufgrund seiner extrem niedrigen Löslichkeit von 2 mg / l in wäßrigen Medien, kann der Wirkstoff nur durch eine spezielle galenische Formulierung in eine zur Erzeugung optimaler Plasmaspiegel nötige Menge in Lösung gebracht werden. Es ist bekannt, daß durch Verarbeitung größerer Hilfsstoffmengen die Löslichkeit einer schwer löslichen Substanz verbessert wird. Da dieses Medikament jedoch zur Langzeittherapie des Bluthochdrucks eingesetzt werden soll, ist es notwendig eine kleine Formulierung mit geringeren Mengen an Hilfsstoffen herzustellen, deren Einnahme problemlos möglich ist.

Die Erfindung betrifft somit eine feste Arzneizubereitung mit hoher Freisetzungsrate enthaltend 1 Gewichtsteil kristallines Nitrendipin, 0,5 - 1 Gewichtsteile Polyvinylpyrrolidon, 0,05 - 0,5 Gewichtsteile Natriumlaurylsulfat, 1 - 3 Gewichtsteile Maisstarke, 0,5 - 2 Gewichtsteile Milchzucker und 0,01 - 0,05 Gewichtsteile Magnesiumstearat.

Bevorzugt kann Polyvinylpynolidon des mittleren Molekulargewichtes von 10 000 - 360 000, insbesondere von 10 000 - 40 000 eingesetzt werden.

Die Arzneizubereitung kann bevorzugt 0,1 - 0,3 Gewichtsteile Natriumlaurylsulfat enthalten.

Die unerwartete Bioverfügbarkeit der erfindungsgemäßen Formulierungen, die zusätzlich den Vorteil der geringen Größe besitzen, ist ersichtlich bei einem Vergleich der Freisetzungsraten von einer erfindungsgemäßen Formulierung (Tablette A, die Natriumlaurylsulphat und Polyvinylpyrrolidon enthält, gegenüber Tabletten B und C wie aus den folgenden Tabellen 1 und 2 entnommen werden kann).

**Tabelle 1**: Zutaten und Gehalt von verschiedenen Tabletten

| | Tablette A (erfindungsgem. Tabl.) | Tablette B | Tablette C |
|---|---|---|---|
| Bay e 5009 | 20,0 mg | 20,0 mg | 20,0 mg |
| Maisstärke | 27,8 mg | 17,5 mg | 167,0 mg |
| Avicel (mikrokristalline Cellulose) | 20,0 mg | 22,6 mg | 48,0 mg |
| Polyvinylpyrrolidon 25 | 10,0 mg | - | - |
| Lactose | - | 16,7 mg | 140,0 mg |
| Natriumaurylsulphat | 2,0 mg | - | - |
| Aerosil | - | 2,0 mg | 4,0 mg |
| Tween 80® | - | 1,0 mg | - |
| Magnesiumstearat | 0,2 mg | 0,2 mg | 1,0 mg |
| Gesamtgewicht | 80,0 mg | 80,0 mg | 380,0 mg |
| Tabletten-Durchmesser | 6,0 mm | 6,0 mm | 10,0 mm |

**Tabelle 2**: Freisetzungsrate (USP-Paddle 4 000 ml; 0,1 n HCl; n = 6)

| Zeit | Tablette A | Tablette B | Tablette C |
|---|---|---|---|
| nach 30 min. | 26,0 % | 5,0 % | 20,0 % |
| nach 60 Min. | 31,0 % | 8,0 % | 23,0 % |
| nach 120 Min. | 32,0 % | 9,0 % | 31,0 % |

Die Herstellung der Arzneizubereitung kann in bekannter Weise durch wäßrige Granulation im Planetenmischer oder im Wirbelbettverfahren oder durch trockene Granulation im Walzverfahren durchgeführt werden. Vorzugsweise wird die wäßrige Granulierung durchgeführt, wobei gegenüber der Granulation im Planetenmischer die Granulation im Wirbelbett über die Sprühung und die Trockenbedingungen besser gesteuert werden kann.

Die Erfindung betrifft auch ein Verfahren zur Herstellung der oben genannten Arzneizubereitung, welches *dadurch gekennzeichnet*, ist, daß man das kristalline Nitrendipin mit Maisstärke und mikrokristalliner Cellulose in Gegenwart einer wäßrigen Lösung aus PVP und Natriumlaurylsulfat granuliert und daß man Magnesiumstearat zufügt und anschließend die Tabletten preßt.

Granulierung im Planetenmischer:

Nitrendipin, Maisstärke, Milchzucker und mikrokristalline Cellulose werden trocken gemischt und mit einer Lösung aus Polyvinylpyrrolidon (PVP), Natriumlaurylsulfat und Wasser granuliert.

Nach Trocknung wird gesiebt und Magnesiumstearat zugesetzt.

**Beispiel 1**

| | |
|---|---|
| Nitrendipin | 20 g |
| Maisstärke | 27 g |
| Milchzucker | 9 g |
| mikrokristalline Cellulose | 14,7g |

werden trocken gemischt und granuliert mit einer Lösung aus

| | |
|---|---|
| PVP (MG 25 000) | 15 g |
| Natriumlaurylsulfat | 4 g |
| Wasser | 10 g |

Die feuchte Masse wird geraspelt, getrocknet und gesiebt. Nach Zusatz von 0,3 g Magnesiumstearat wird das Granulat zu Tabletten verpreßt, die 20 mg Nitrendipin enthalten.

**Beispiel 2**

| | |
|---|---|
| Nitrendipin | 10 g |
| Maisstärke | 23 g |
| Milchzucker | 15 g |
| mikrokristalline Cellulose | 14,7 g |

werden trocken gemischt und granuliert mit einer Lösung aus

| | |
|---|---|
| PVP (MG 25 000) | 10 g |
| Natriumlaurylsulfat | 2 g |
| Wasser | 10 g |

Die feuchte Masse wird geraspelt, getrocknet und gesiebt. Nach Zusatz von 0,3 g Magnesiumstearat wird das Granulat zu Tabletten enthaltend 10 mg Nitrendipin verpreßt.

Granulierung im Wirbelbett:

Nitrendipin, Maisstärke, Milchzucker und mikrokristalline Cellulose werden im Wirbelbett gemischt und im Sprühverfahren mit einer Lösung, bestehend aus Polyvinylpyrrolidon, Natriumlaurylsulfat und Wasser granuliert.

Nach der Trocknung wird gesiebt und Magnesiumstearat zugesetzt.

**Beispiel 3**

| | |
|---|---|
| Nitrendipin | 20 g |
| Maisstärke | 25 g |
| Milchzucker | 6 g |
| mikrokristalline Cellulose | 14,7 g |

werden trocken gemischt und granuliert mit einer Lösung aus

| | |
|---|---|
| PVP (MG 10 000) | 20 g |
| Natriumlaurylsulfat | 4 g |
| Wasser | 10 g |

Die feuchte Masse wird geraspelt, getrocknet und gesiebt. Nach Zusatz von 0,3 g Magnesiumstearat wird das Granulat zu Tabletten verpreßt, die 20 mg Nitrendipin enthalten.

**Beispiel 4**

| | |
|---|---|
| Nitrendipin | 20 g |
| Maisstärke | 25 g |
| Milchzucker | 6 g |
| mikrokristalline Cellulose | 14,7 g |

werden im Wirbelbett trocken gemischt und mit einer Lösung aus

| | |
|---|---|
| PVP (MG 25 000) | 20 g |
| Natriumlaurylsulfat | 4 g |
| Wasser | 10 g |

im Sprühverfahren granuliert. Nach Beendigung des Sprühvorgangs wird getrocknet und gesiebt. Nach Zugabe von 0,3 g Magnesiumstearat wird das fertige Granulat zu Tabletten enthaltend 20 mg Nitrendipin verpreßt.

**Beispiel 5**

| | |
|---|---|
| Nitrendipin | 10 g |
| Maisstärke | 23 g |
| Milchzucker | 15 g |
| mikrokristalline Cellulose | 14,7 g |

werden im Wirbelbett gemischt und eine Lösung aus

| | |
|---|---|
| PVP (MG 25 000) | 10 g |
| Natriumlaurylsulfat | 2 g |
| Wasser | 10 g |

eingerührt. Die Mischung wird getrocknet und gesiebt. Nach Zugabe von 0,3 g Magnesiumstearat werden Tabletten enthaltend 10 mg Nitrendipin gepreßt.

**Beispiel 6**

| | |
|---|---|
| Nitrendipin | 10 g |
| Maisstärke | 26 g |
| Milchzucker | 17 g |
| mikrokristalline Cellulose | 14,7 g |

werden im Wirbelbett gemischt und eine Lösung aus

| | |
|---|---|
| PVP (MG 40 000) | 5 g |
| Natriumlaurylsulfat | 2 g |
| Wasser | 10 g |

eingerührt. Die Mischung wird getrocknet und gesiebt. Nach Zugabe von 0,3 g Magnesiumstearat werden Tabletten enthaltend 10 mg Nitrendipin gepreßt.

**Patentansprüche**

1. Feste Arzneizubereitung mit hoher Freisetzungsrate enthaltend 1 Gewichtsteil kristallines Nitrendipin, 0,5 - 1 Gewichtsteile Polyvinylpyrrolidon, 0,05 0,5 Gewichtsteile Natriumlaurylsulfat, 1 - 3 Gewichtsteile Maisstärke, 0,5 - 2 Gewichtsteile mikrokristalline Cellulose, 0 - 2 Gewichtsteile Milchzucker und 0,01 - 0,05 Gewichtsteile Magnesiumstearat.

2. Feste Arzneimittelzubereitung gemäß Anspruch 1 enthaltend 0,1 - 0,3 Gewichtsteile Natriumlaurylsulfat.

3. Feste Arzneimittelzubereitung gemäß Anspruch 1 enthaltend Polyvinylpyrrolidon eines mitteleren Molekulargewichtes von 10 000 - 360 000.

4. Feste Arzneimittelzubereitung gemäß Anspruch 1 enthaltend Polyvinylpyrrolidon eines mittleren Molekulargewichts von 10 000 - 40 000.

5. Verfahren zur Herstellung einer festen Arzneizubereitung mit hoher Freisetzungsrate enthaltend 1 Gewichtsteil kristallines Nitrendipin, 0,5 - 1 Gewichtsteile Polyvinylpyrrolidon, 0,05 - 0,5 Gewichtsteile Natriumlaurylsulfat, 1 - 3 Gewichtsteile Maisstärke, 0,5 - 2 Gewichtsteile mikrokristalline Cellulose, 0 - 2 Gewichtsteile Milchzucker und 0,01 0,05 Gewichtsteile Magnesiumstearat, *dadurch gekennzeichnet*, daß man das kristalline Nitrendipin mit der oben genannten Maisstärke, der mikrokristallinen Cellulose, in Gegenwart einer wäßrigen Lösung aus PVP und Natriumlaurylsulfat granuliert und das Magnesiumstearat vor dem Tablettenpressen beifügt.

**Claims**

1. Solid medicament formulation with a high release rate, containing 1 part by weight of crystalline nitrendipine, 0.5 - 1 part by weight of polyvinyl-pyrrolidone, 0.05 0.5 part by weight of sodium lauryl sulphate, 1 - 3 parts by weight of maize starch, 0.5 - 2 parts by weight of microcrystalline cellulose, 0 - 2 parts by weight of lactose and 0.01 - 0.05 part by weight of magnesium stearate.

2. Solid medicament formulation according to Claim 1, containing 0.1 - 0.3 part by weight of sodium lauryl sulphate.

3. Solid medicament formulation according to Claim 1, containing polyvinylpyrrolidone with an average molecular weight of 10 000 - 360 000.

4. Solid medicament formulation according to Claim 1, containing polyvinylpyrrolidone with an average molecular weight of 10 000 - 40 000.

5. Process for the preparation of a solid medicament formulation with a high release rate, containing 1 part by weight of

crystalline nitrendipine, 0.5 - 1 part by weight of polyvinylpyrrolidone, 0.05 - 0.5 part by weight of sodium lauryl sulphate, 1 - 3 parts by weight of maize starch, 0.5 - 2 parts by weight of microcrystalline cellulose, 0 - 2 parts by weight of lactose and 0.01 - 0.05 part by weight of magnesium stearate, *characterised* in that the crystalline nitrendipine is ganulated with the abovementioned maize starch and with thę microcrystalline cellulose, in the presence of an aqueous solution of PVP and sodium lauryl sulphate, and the magnesium stearte is added before pressing the tablets.

**Revendications**

1. Préparation pharmaceutique solide à grande vitesse de libération, contenant 1 partie en poids de nitrendipine cristalline, 0,5 à 1 partie en poids de polyvinylpyrrolidone, 0,05 à 0,5 partie en poids de laurylsulfate de sodium, 1 à 3 parties en poids d'amidon de maïs, 0,5 à 2 parties en poids de cellulose microcristalline, 0 à 2 parties en poids de lactose et 0,01 à 0,05 partie en poids de stéarate de magnésium.

2. Préparation pharmaceutique solide suivant la revendication 1, contenant 0,1 à 0,3 partie en poids de laurylsulfate de sodium.

3. Préparation pharmaceutique solide suivant la revendication 1, contenant de la polyvinylpyrrolidone de poids moléculaire moyen allant de 10 000 à 360 000.

4. Préparation pharmaceutique solide suivant la revendication 1, contenant de la polyvinylpyrrolidone de poids moléculaire moyen allant de 10 000 à 40 000.

5. Procédé de production d'une préparation pharmaceutique solide à grande vitesse de libération contenant 1 partie en poids de nitrendipine cristalline, 0,5 à 1 partie en poids de polyvinylpyrrolidone, 0,05 à 0,5 partie en poids de laurylsulfate de sodium, 1 à 3 parties en poids d'amidon de maïs, 0,5 à 2 parties en poids de cellulose microcristalline, 0 à 2 parties en poids de lactose et 0,01 à 0,05 partie en poids de stéarate de magnésium, *caractérisé* en ce qu'on granule la nitrendipine cristalline avec l'amidon de maïs mentionné ci-dessus et la cellulose microcristalline en présence d'une solution aqueuse de PVP et de laurylsulfate de sodium, et on ajoute le stéarate de magnésium avant le pressage en comprimés.